# EUROPEAN PATENT APPLICATION

(11) **EP 1 524 522 A1**
(43) Date of publication of application: **20.04.2005**
(21) Application number: 04256314.8
(22) Date of filing: 13.10.2004
(51) Int. Cl.: G01N 33/497, G01N 33/00

(54) **Apparatus for measuring carbon monoxide on a person's breath**

(30) Priority: 17.10.2003 GB 0324366
(71) Applicant: Micro Medical Limited, Rochester, Kent ME1 2AZ (GB)
(72) Inventor: Lawson, Christopher Patrick, Rochester Kent ME1 2BN (NL)
(74) Representative: Jones, Graham H.

(57) **Abstract**

Apparatus (2) for measuring carbon monoxide on a person's breath, which apparatus (2) comprises measuring means (4) for measuring the carbon monoxide on the person's breath, and message giving means (6) for giving a message which is in a language and which is appropriate to the amount of the carbon monoxide measured on the person's breath.

## Description

This invention relates to apparatus for measuring carbon monoxide on a person's breath.

Apparatus for measuring carbon monoxide on a person's breath is known. The known apparatus comprises measuring means for measuring the carbon monoxide on the person's breath, and result giving means for giving the result of the measured carbon monoxide on the person's breath. The measuring means is usually an electrochemical fuel cell which measures the concentration of the carbon monoxide on the person's breath. The result giving means is usually in the form of a display of the concentration in parts per million and/or a plurality of lights, with one of the lights becoming illuminated as appropriate to the measured concentration of the carbon monoxide on the person's breath. The result is usually explained to the person by a doctor, clinician, nurse or other person conducting the measurement. The measurement is usually explained in terms of the damage that is being done to the person's body. This is in order to discourage the person from smoking. The measurement may also be used to monitor the effectiveness of any smoking cessation program for the person. The apparatus is not always as effective in discouraging smoking as would be preferred.

It is an aim of the present invention to reduce the above mentioned problem.

Accordingly, in one non-limiting embodiment of the present invention there. is provided apparatus for measuring carbon monoxide on a person's breath, which apparatus comprises measuring means for measuring the carbon monoxide on the person's breath, and message giving means for giving a message which is in a language and which is appropriate to the amount of the carbon monoxide measured on the person's breath.

Because the apparatus of the present invention gives a message which is in a language, this message is able to be clearly understood by all person's having their breath measured. It is not necessary for the measurement to be explained by a doctor, clinician, nurse or other person conducting the measurement. The apparatus can even be used by the person on their own since the message does not have to be interpreted and explained. Because the message is in a language, the message is more direct and is likely to have a more positive effect in encouraging the person to stop smoking.

Preferably, the apparatus is one in which the message giving means is an audio message giving means for giving the message as an audio message. With such a message giving means, the message is not only in a language which is thus easily able to be understood, but the message is also audible and which is believed to be the best way of giving the person the message with a view to getting the person to cease smoking.

Alternatively, or in addition, as may be desired, the message giving means may be a visual message giving means for giving the message as a visual message.

The apparatus may be one in which the message giving means selects an appropriate message from a plurality of messages contained in the apparatus.

The plurality of messages may be interpretive messages. In this case, the plurality of interpretive messages may be as follows:
(i) **Non-smoker** - this message being for a measured concentration of carbon monoxide of 0 - 6 ppm (parts per million).
(ii) **Light-smoker** - this message being for a measured concentration of carbon monoxide of 7 - 10 ppm.
(iii) **Heavy-smoker** - this message being for a measured concentration of carbon monoxide of 11 or more ppm.

The above messages are examples of messages that may be employed. Thus, for example, different messages from a choice of three messages may be employed, or there may be more than three messages to choose from.

Usually as an alternative to the interpretive messages, but if desired in addition to the interpretive messages, the plurality of messages may be prescriptive messages. In this case, the plurality of prescriptive messages may be as follows:
(i) **No therapy required -** this message being for a measured concentration of carbon monoxide of 0 - 6 ppm.
(ii) **Low dosage nicotine replacement recommended** - this message being for a measured concentration of carbon monoxide of 7 - 10 ppm.
(iii) **High dosage nicotine replacement therapy recommended**
   - this message being for a measured concentration of carbon monoxide of 11 or more ppm.

The above messages are examples of messages that may be employed. Thus, for example, different messages from a choice of three messages may be employed, or there may be more than three messages to choose from.

Usually as an alternative to the interpretive messages or the prescriptive messages, the plurality of messages may be disincentive messages. If desired however the disincentive messages may be in addition to the interpretive messages and/or the prescriptive messages. The plurality of disincentive messages may be as follows:
(i) **Sweet breath** - this message being for a measured concentration of carbon monoxide of 0 - 6 ppm.
(ii) Breath stinks, recommend you give up smoking - this message being for a measured concentration of carbon monoxide of 7 - 10 ppm.
(iii) Breath stinks really badly, recommend you give up smoking immediately - this message being for a measured concentration of carbon monoxide of 11 or more ppm.

The apparatus of the present invention may be used such that it only comprises the measuring means and the message giving means. If desired however the apparatus of the present invention can be used with result giving means of the type currently used in known apparatus and as mentioned above. In this case, the apparatus of the present invention may include result giving means for giving the result of the measured carbon monoxide on the person's breath, the result giving means being such that it gives the result in a form which is not in a language, and which is in a form which is only as a visual display.

The result may be given in a form which would not normally be understood by the person, and which would normally need to be explained to the person by another person who would be in charge of the measurement. As indicated above, such a person might be a doctor, clinician or a nurse. The result may be given as a measured concentration of carbon monoxide in parts per million.

The result giving means may select an appropriate result from a plurality of results contained in the apparatus, and may light up one of a plurality of different lights. The plurality of results and the plurality of different lights may be as follows:
(i) **Non-smoker** - green light
(ii) **Light-smoker** - orange light
(iii) **Heavy-smoker** - red light

The apparatus of the present invention may include a display panel for displaying visual messages.

The apparatus of the present invention may be one in which the measuring means is an electrochemical fuel cell. Other types of measuring means may be employed if desired.

The apparatus is preferably one which is portable. The apparatus may however be static if desired. Preferably the portable apparatus is hand holdable.

An embodiment of the invention will now be described solely by way of example and with reference to the accompanying drawings in which:
Figure 1 is a perspective view of apparatus for measuring carbon monoxide on a person's breath; and
Figure 2 shows in block diagram form the apparatus shown in Figure 1.

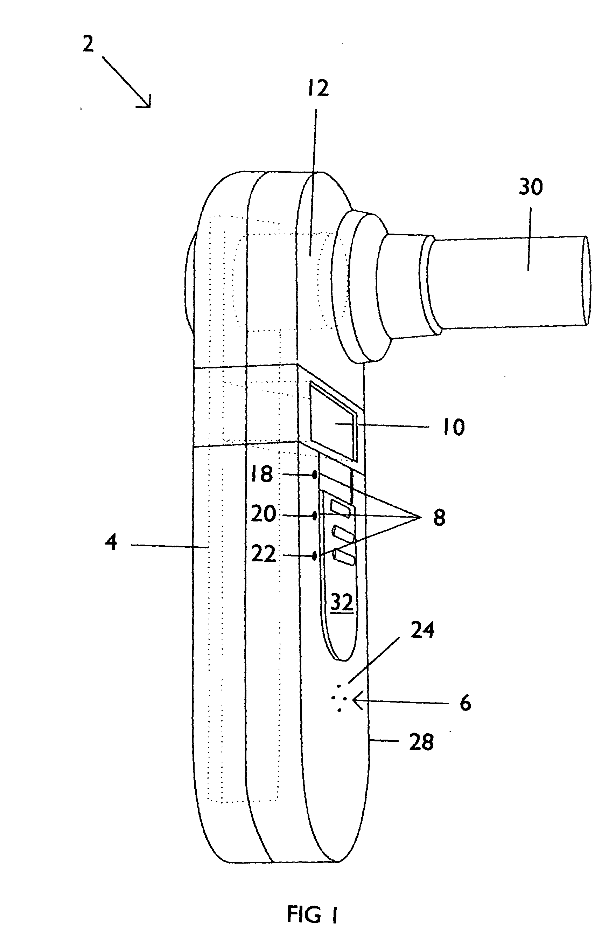

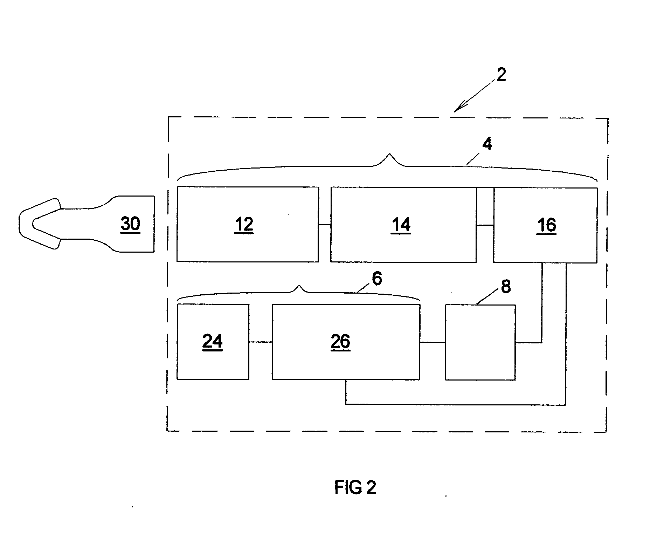

Referring to Figures 1 and 2, there is shown apparatus 2 for measuring carbon monoxide on a person's breath. The apparatus 2 comprises measuring means 4 for measuring the carbon monoxide on the person's breath. The apparatus 2 also comprises message giving means 6. The message giving means 6 is for giving a message which is in a language and which is appropriate to the amount of the carbon monoxide measured on the person's breath. More specifically, the message giving means 6 is an audio message giving means 6 for giving the message as an audio message.

The message giving means 6 selects an appropriate message from a plurality of messages contained in the apparatus 2. The plurality of messages will usually be either interpretive messages, prescriptive messages or disincentive messages. Examples of these different types of messages are given herein above.

The apparatus 2 also includes result giving means 8 for giving the result of the measured carbon monoxide on the person's breath. The result giving means 8 is such that it gives the result in a form which is not in a language, and which is in a form which is only as a visual display. More specifically, the result giving means 8 gives the result as a measured concentration of carbon monoxide in parts per million. The result giving means 8 may select an appropriate result from a plurality of results contained in the apparatus 2 and may light up one of a plurality of different lights. Examples of such results and lights are given herein above.

As can best be seen from Figure 1, the apparatus 2 includes a display panel 10 for displaying visual messages.

The measuring means 4 shown in Figure 1 has simply been shown as a printed circuit board 4. The measuring means 4 will also include a carbon monoxide sensor 12 as shown in Figure 2. The carbon monoxide sensor 12 may be an electrochemical fuel cell or any other suitable and appropriate device. As shown in Figure 2, the measuring means 4 includes a signal conditioning circuit 14 and a processor control circuit 16.

The result giving means 8 is shown in Figure 1 as including three lights 18, 20, 22. The light 18 may be a green light for indicating a non-smoker. The light 20 may be an orange light for indicating a light-smoker. The light 22 may be a red light for indicating a heavy-smoker.

As shown in Figure 2, the message giving means 6 comprises a speaker 24 and a recorded messages storage device 26. The recorded messages storage device 26 may include messages such for example as those given herein above.

The apparatus 2 comprises a body 28 and a mouthpiece 30. A person using the apparatus 2 blows into the mouthpiece 30. As can be seen from Figure 1, the carbon monoxide sensor 12 is positioned in line with the mouthpiece 30 in order to receive the breath exhaled by the person.

During use of the apparatus 2, the apparatus 2 is turned on via a switch 32 and then a person exhales through the mouthpiece 4. The exhaled breath is directed over the carbon monoxide sensor 12. An electrical output from the carbon monoxide sensor 12 is processed by the signal conditioning circuit 14, and is turned into a digital format to be read by a processor forming part of the processor control circuit 16. The processor calculates the exhaled carbon monoxide concentration, and displays the result on the display panel 10. The processor also sends a signal to the recorded messages storage device 26, in order to cause an appropriate message to be sent to the speaker 24.

It is to be appreciated that the embodiment of the invention described above with reference to the accompanying drawings has been given by way of example only and that modifications may be effected. Thus, for example, the apparatus 2 may be of a different design to that shown in Figure 1.

## Claims

1. Apparatus for measuring carbon monoxide on a person's breath, which apparatus comprises measuring means for measuring the carbon monoxide on the person's breath, and message giving means for giving a message which is in a language and which is appropriate to the amount of the carbon monoxide measured on the person's breath.

2. Apparatus according to claim 1 in which the message giving means is an audio giving means for giving the message as an audio message.

3. Apparatus according to claim 1 or claim 2 in which the message giving means is a visual message giving means for giving the message as a visual message.

4. Apparatus according to any one of the preceding claims in which the message giving means selects an appropriate message from a plurality of messages contained in the apparatus.

5. Apparatus according to claim 4 in which the plurality of messages are interpretive messages.

6. Apparatus according to claim 5 in which the plurality of interpretive messages are as follows:
(i) **Non-smoker** - this message being for a measured concentration of carbon monoxide of 0 - 6 ppm.
(ii) **Light-smoker** - this message being for a measured concentration of carbon monoxide of 7 - 10 ppm.
(iii) **Heavy-smoker** - this message being for a measured concentration of carbon monoxide of 11 or more ppm.

7. Apparatus according to any one of claims 4 - 6 in which the plurality of messages are prescriptive messages.

8. Apparatus according to claim 7 in which the plurality of prescriptive messages are as follows:
(i) **No therapy required** - this message being for a measured concentration of carbon monoxide of 0 - 6 ppm.
(ii) **Low dosage nicotine replacement recommended** - this message being for a measured concentration of carbon monoxide of 7 - 10 ppm.
(iii) **High dosage nicotine replacement therapy recommended**
- this message being for a measured concentration of carbon monoxide of 11 or more ppm.

9. Apparatus according to any one of claims 4 - 8 in which the plurality of messages are disincentive messages.

10. Apparatus according to claim 9 in which the plurality of disincentive messages are as follows:
(i) **Sweet breath -** this message being for a measured concentration of carbon monoxide of 0 - 6 ppm.
(ii) **Breath stinks, recommend you give up smoking** - this message being for a measured concentration of carbon monoxide of 7 - 10 ppm.
(iii) **Breath stinks really badly, recommend you give up smoking immediately** - this message being for a measured concentration of carbon monoxide of 11 or more ppm.

11. Apparatus according to any one of the preceding claims and including result giving means for giving the result of the measured carbon monoxide on the person's breath, the result giving means being such that it gives the result in a form which is not in a language, and which is in a form which is only as a visual display.

12. Apparatus according to claim 11 in which the result is given in a form which would not normally be understood by the person, and which would normally need to be explained to the person by another person who would be in charge of the measurement.

13. Apparatus according to claim 12 in which the result is given as a measured concentration of carbon monoxide in parts per million.

14. Apparatus according to any one of claims 11 - 13 in which the result giving means selects an appropriate result from a plurality of results contained in the apparatus, and lights up one of a plurality of different lights.

15. Apparatus according to claim 14 in which the plurality of results and the plurality of different lights are as follows:
(i) **Non-smoker** - green light
(ii) **Light-smoker** - orange light
(iii) **Heavy-smoker** - red light

16. Apparatus according to any one of the preceding claims and including a display panel for displaying visual messages.

17. Apparatus according to any one of the preceding claims in which the measuring means is an electrochemical fuel cell.

18. Apparatus according to any one of the preceding claims and which is portable.

19. Apparatus according to claim 18 and which is hand holdable.
